# EUROPEAN PATENT APPLICATION

(11) **EP 1 201 761 A1**
(43) Date of publication of application: **02.05.2002**
(21) Application number: 00946382.9
(22) Date of filing: 18.07.2000
(51) Int. Cl.: C12N 15/861, C12N 5/10

(54) **METHOD OF CONSTRUCTING RECOMBINANT ADENOVIRUS VECTOR**

(30) Priority: 19.07.1999 JP 20535599
(71) Applicant: Japan Science and Technology Corporation, Kawaguchi-shi, Saitama 332-0012 (JP)
(72) Inventor: MIYAZAKI, Junichi, Suita-shi, Osaka 565-0872 (JP); TASHIRO, Fumi, Ibaraki-shi, Osaka 567-0057 (JP)
(74) Representative: Dzieglewska, Hanna Eva
(86) International application number: JP0004815
(87) International publication number: WO0105989

(57) **Abstract**

A recombinant adenovirus vector comprising an adenovirus genome DNA and an expression cassette is produced by inserting and ligating a cosmid sequence having recombinase recognition sequences at both ends and the expression cassette into either deletion sites of the adenovirus genome DNA with the deletion of the E1 region or E1 and E3 regions to thereby construct a recombinant cosmid/adenovirus vector; cotransfecting this recombinant cosmid/adenovirus vector and a recombinase-expression vector into a cell line producing adenovirus E1 protein; and deleting the cosmid vector sequence from the recombinant cosmid/adenovirus vector in the cells. This method makes it possible to conveniently and efficiently construct a recombinant adenovirus vector.

## Description

### Technical Filed

The invention of the present application relates to a method for constructing a recombinant adenovirus vector. More specifically, the invention of the present application relates to a method for constructing a recombinant adenovirus vector which is useful for introducing genes into mammalian cells, and genetic engineering materials for said method.

### Background Art

In recent years, the life science has made a long stride progress due to the accumulation of knowledge and the development of various technology in the fields of molecular biology, molecular genetics and the like, whereby a number of information for life phenomena can now be obtained. Research and development are being vigorously carried out in various fields, and the analysis of gene functions has a significant weight among such research and development Specifically, various technology for introducing an isolated gene into cells or organs in vivo, and vectors which are used for such technology, have been developed.

Vectors of a number of types which are used for introducing genes to a mammalian cell have also been developed. In recent years, vectors produced by utilizing virus (virus vectors) are drawing attention. Among the virus vectors, an adenovirus vector, in particular, can be infected not only to dividing cells but also to non-dividing cells, thereby can be prepared at a high virus titer. In addition, as an adenovirus vector can express the targeted gene under the control of enhancers and promoters of various combinations, the medical application of the adenovirus vector to gene therapy have been studied.

Up to now, a number of methods of constructing a recombinant adenovirus vector, such as a method of utilizing in vivo homologous recombination and a method by in vitro ligation, have been developed. When a recombinant adenovirus vector having the adenovirus genome in which the E1 region has been deleted is used, the introduced gene is expressed but the infectious viral particles are not produced. Therefore, a recombinant adenovirus vector is utilized especially as a useful means for introducing genes in gene therapy or the like.

Adenovirus vector has a number of advantages as compared with other virus vectors, as described above. However, as the virus genome of an adenovirus vector is relatively long (36 kb), there arise some problems, in actual construction of a recombinant vector, such as the complicated procedures or the low efficiency in construction processes. Examples of a method of constructing a recombinant adenovirus vector in which the E1 region has been replaced include a method of utilizing in vivo homologous recombination in a mammalian cell (the 293 cell, for example) between two plasmids (e.g., Virology 163:614-617, 1988; Nucleic Acids Res. 26:3687-3693, 1998). In such a method, the first plasmid includes the 5' end ITR (inverted terminal repeat) of the adenovirus genome, the packaging signal, the target gene and any selected portion of the virus genome. The second plasmid includes the virus genome portion which overlaps the first plasmid and the remaining portion of the virus genome which contains the 3' end ITR. However, as the possibility that the homologous recombination occurs in a mammalian cell is quite low, in the method described above, it is necessary to carry out transfection of both of the plasmids to a mammalian cell on a large scale.

Alternatively, as another method, the COS-TPC method has been proposed (Proc. Natl. Acad. Sci. USA 93:1320-1324, 1996). This method includes the step of transfecting a mammalian cell with a cosmid vector containing an adenovirus genome of whole length together with an expression unit of the targeted gene, and the virus DNA-TPC (terminal protein complex) which has been subjected to digestion by a restriction enzyme. In this method, by using the virus DNA-TPC, the target recombinant adenovirus vector can be efficiently retrieved. However, in the case of this method, the elimination of the parental adenovirus is insufficient, although a measure for preventing the parental adenovirus from appearing is taken by digesting the adenovirus genome by the restriction enzyme. In short, this method has a problem in terms of safety.

As a yet another method, there has been reported a method of constructing a recombinant adenovirus vector to which the Cre-loxP recombinant system is applied (J. Virol. 71:1842-1849, 1997). In this method, by the action of the Cre recombinase, a recombination between molecules (specifically, a recombination between the adenovirus as the donor and the shuttle plasmid having the expression cassette) is effected and a recombinant adenovirus vector can be constructed efficiently. However, in the case of this invention, donor virus must be prepared and there is a possibility that the donor virus remains in the final virus sample.
Further, there has been an attempt to reconstruct the whole adenovirus genome, in vitro, with using a cosmid vector. In this case, however, the complicated DNA constructing process is required prior to the transfection to a mammalian cell line, which is not practical.

### Problems to be solved by the present Invention:

Prior to the aforementioned various reports and proposals regarding the method of constructing a recombinant adenovirus vector, Graham reported that, when a circular DNA constructed by inserting a small plasmid at the restriction enzyme Xba I site, which site exists at the one location in the E1 region of the adenovirus 5 type, is transfected to a mammalian cell line (the 293 cells), the circular DNA produces the infectious virus as efficiently as the virus DNA does (EMBO J. 3:2917-2922, 1984).

This report suggests that a recombinant adenovirus vector can be easily constructed by replacing the E1 region or E3 region of a circular adenovirus DNA with an exogenous gene. However, when a recombinant adenovirus vector is actually constructed according to this method, there arise two problems. One is the low efficiency in incorporating the expression cassette into the extremely large plasmid which contains the adenovirus genome DNA. The other problem is that the plasmid DNA portion remains in the constructed adenovirus vector.

### Disclosure of Invention

The invention of the present application has an object to provide a novel method of easily and efficiently constructing a recombinant adenovirus vector, and genetic engineering materials for implementing the method invention.

The first aspect of the present invention is a method for constructing a recombinant adenovirus vector having a DNA sequence consisting of an adenovirus genome DNA and an expression cassette, which comprises:
constructing a recombinant cosmid/adenovirus vector by inserting and ligating a cosmid sequence having recombinase recognition sequences at both ends and the expression cassette into a site of the adenovirus genome DNA where E1 region or E1 and E3 regions are deleted;
cotransfecting this recombinant cosmid/adenovirus vector and a recombinase-expression vector into a cell line producing adenovirus E1 protein; and
deleting the cosmid vector sequence from the recombinant cosmid/adenovirus vector in the cells.

In the method according to the first aspect of the present invention, it is preferable that the recombinase is Cre recombinase and the recognition sequences thereof are loxP sequences, or the recombinase is FLP recombinase and the recognition sequences thereof are FRT sequences. In addition, it is preferable that the cell producing adenovirus E1 protein is 293 cell derived from human fetal kidney cells.

The second aspect of the present invention is a method for constructing a recombinant adenovirus vector having a DNA sequence consisting of an adenovirus genome DNA and an expression cassette, which comprises:
constructing a recombinant cosmid/adenovirus vector by inserting and ligating a cosmid sequence having recombinase recognition sequences at both ends and the expression cassette into a site of the adenovirus genome DNA where E1 region or E1 and E3 regions are deleted;
transfecting this recombinant cosmid/adenovirus vector into a cell line producing recombinase and adenovirus E1 protein; and
deleting the cosmid vector sequence from the recombinant cosmid/adenovirus vector in the cells.

In the method according to the second aspect of the present invention, it is preferable that the recombinase is Cre recombinase and the recognition sequences thereof are loxP sequences or the recombinase is FLP recombinase and the recognition sequences thereof are FRT sequences. In addition, it is preferable that the cell producing the recombinase and adenovirus E1 protein is the 293 cell derived from human fetal kidney cells which produces the recombinase.

The third aspect of the present invention is a cosmid/adenovirus vector, which comprises a cosmid sequence having recombinase recognition sequences at both ends in a site of the adenovirus genome DNA where E1 region or E1 and E3 regions are deleted.

In the cosmid/adenovirus vector of the third aspect of the present invention, it is preferable that the recombinase is Cre recombinase and the recognition sequences thereof are loxP sequences, or the recombinase is FLP recombinase and the recognition sequences thereof are FRT sequences.

The fourth aspect of the present invention is a 293 cell line derived from human fetal kidney cells which produce FLP recombinase.

Specifically, in the aforementioned aspects of the present invention, "a cosmid sequence" means a linear cosmid vector sequence having recombinase recognition sequences in the same direction at both ends. In addition, "a cosmid/adenovirus vector" means a circular DNA construct which comprises said cosmid sequence and the adenovirus genome DNA with the deletion of the E1 region or E1 and E3 regions. Further, "a recombinant cosmid/adenovirus vector" means a circular DNA construct produced by incorporating an expression cassette into said cosmid/adenovirus vector. Yet further, "a recombinant adenovirus vector" means an adenovirus including a linear DNA sequence produced by removing the cosmid sequence from said recombinant cosmid/adenovirus vector in the infectious viral particle.

### Brief Description of Drawings

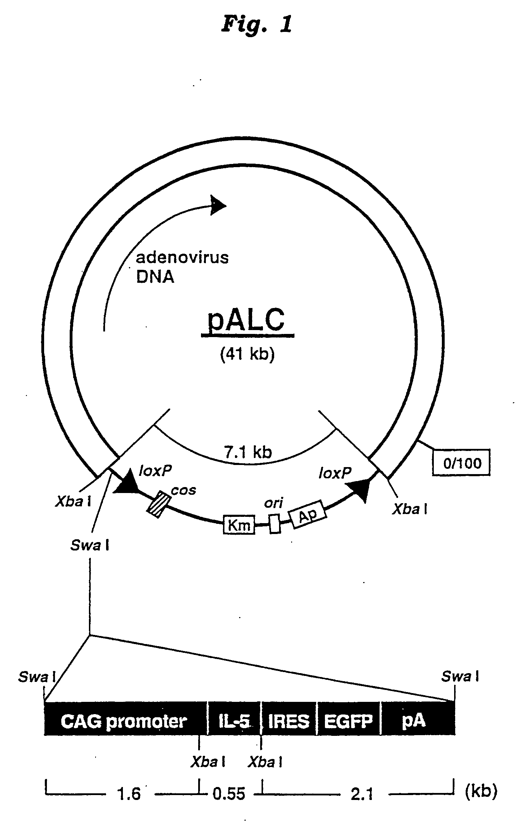

Fig. 1 is a schematic view which shows structures of a cosmid/adenovirus vector pALC (upper) and an expression cassette (lower) constructed in example 1. The adenovirus genome DNA of 34 kb is shown as a white ring and the direction of the genome is indicated by the arrow. The cosmid vector of 7 kb is interposed by the loxP sequences shown by the black triangles, and includes therein the cos site, the kanamycin resistance gene (Km), the origin of replication (ori) that works in Escherichia coli and the ampicillin resistance gene (Ap). The expression cassette consists of the CAG promoter, the mouse IL-5 cDNA, IRES, EGFP cDNA, and the poly(A) additional signal (pA). The expression cassette is inserted into the Swa I site of the pALC vector, whereby a recombinant cosmid/adenovirus vector pALC-IL-5 of example 2 is constructed.

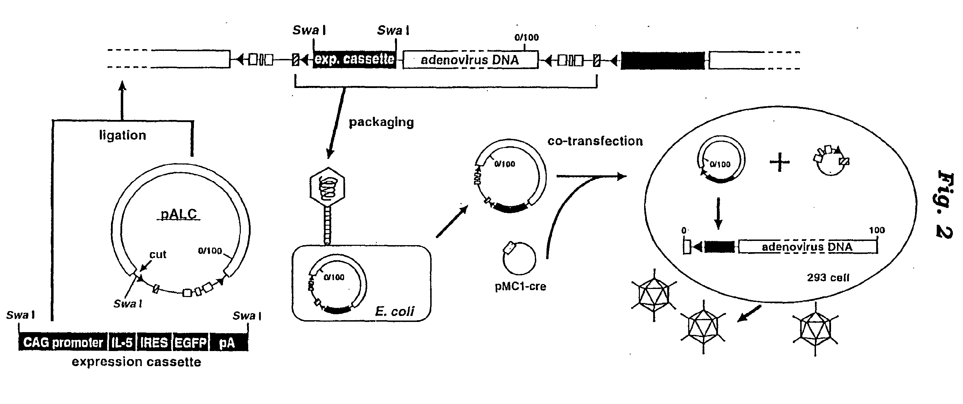

Fig. 2 is a schematic view which shows the main steps of processing the recombinant adenovirus vector constructed in example 3. The pALC-IL-5 of example 2 was amplified in Escherichia coli DH10B, and the amplified pALC-IL-5 was purified. The 293 cells were transfected with the purified pALC-IL-5 and the pMC1-cre plasmid. The Cre recombinase, which expresses itself in a transient manner, cuts out the cosmid sequence of 7 kb efficiently, whereby an infectious recombinant adenovirus particles containing the expression cassette of IL-5 and EGFP were eventually produced.

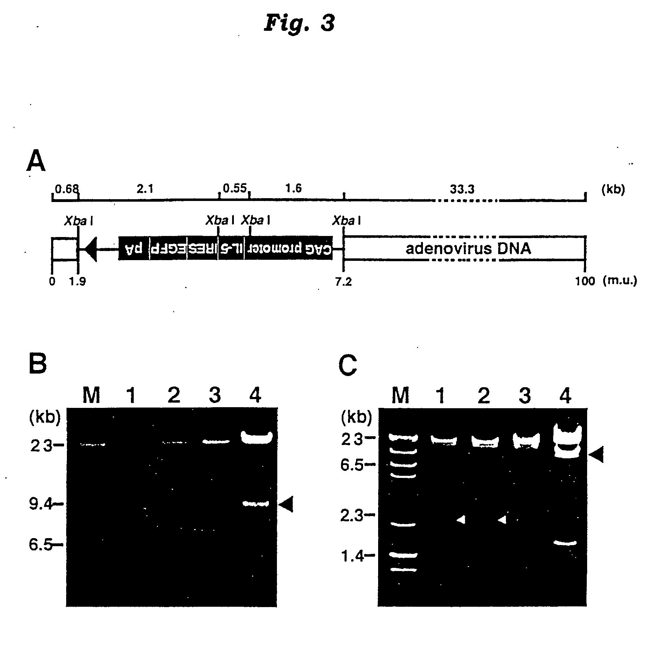

Fig. 3A is a schematic view which shows a structure of the recombinant adenovirus vector DNA constructed in example 3. One loxP (indicated by the black triangle in the drawing) of the cosmid sequence which has been removed by the Cre recombinase remains in the ligated structure of the adenovirus genome (indicated as the open bar in the drawing) and the expression cassette of IL-5 and EGFP (indicated by the black bar in the drawing). The numbers indicated on the lower row of Fig. 3A represent the map unit (m.u.) of the adenovirus genome. The numbers on the upper row of Fig. 3A each represent the lengths (kb) of the fragments when the recombinant adenovirus vector DNA is digested with Xba I. Fig. 3B and Fig. 3C are photographs, each showing the pattern observed when various DNA samples were digested with Xba I and subjected to electrophoresis in 0.4 % (B) or 0.9 % (C) agarose gel. Specifically, lane 1 and lane 2 represent the virus DNA produced by cotransfecting the 293 cell line with pALC-IL-5 and pMC1-cre; lane 3 represents the virus DNA produced by transfecting the 293 cell line with pFG140; and lane 4 represents pALC-IL-5DNA. The DNA of the lane 1 and that of lane 2 were each prepared from the virus stocks which had been obtained by different transfection processes performed independent of each other. The cosmid sequence interposed by the loxP sites is contained in the DNA fragment of 9.1 kb (lane 4, the black triangle). In the recombinant adenovirus DNA, no fragment of 9.1 kb was observed but a fragment of 2.1 kb was generated (lanes 1 and 2, the white triangle). The lane M represents the electrophoresis pattern of the size marker and the size thereof is indicated on the left-side end (kb).

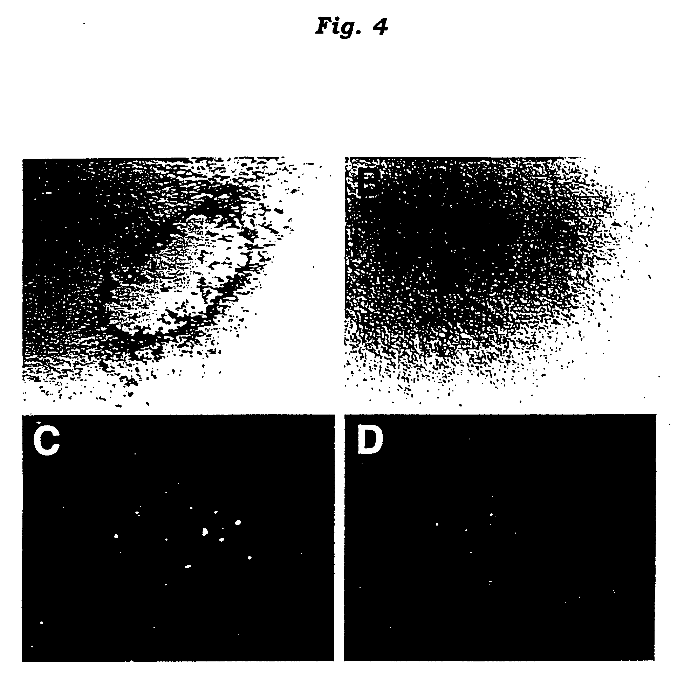

Figs. 4A-4D are photographs each showing the plaque formation and the expression of EGFP in the vicinity of the plaque, of the recombinant adenovirus vector constructed by example 3. In the 293 cell line which had been cotransfected with pALC-IL-5 and pMC1-cre, plaques formed by the virus were observed (Figs. 4A and 4C). Similarly, in the 293 cell line which had been infected with the recombinant adenovirus vector, plaques were observed (Figs. 4B and 4D). When the fields of the photographs A and B were observed with a fluorescent microscope, fluorescent light emission of EGFP was confirmed (Figs. 4C and 4D).

### Best Mode for Carrying Out the Invention

In the methods for constructing a recombinant adenovirus vector of the first and the second aspects of the present invention, the cosmid/adenovirus vector of the third aspect of the present invention is used. In the preparation of the cosmid/adenovirus vector, known clones (such as pFG140 or the like used in the Examples) may be employed as the adenovirus genome DNA (36 kb, approximately). By treating such genome DNA with an appropriate restriction enzyme, nuclease or the like, DNA fragments (31-34 kb, approximately) with the deletion of the E1 region (2.0 kb, approximately) or both E1 and E3 regions (3.0 kb, approximately) can be obtained. This DNA fragment is ligated with a cosmid sequence which has been cut and linearized, whereby a circular DNA construct is formed. As the resulting cosmid vector is a plasmid having the cos site of Escherichia coli λ phage and exogenous DNA of 30-42 kb can be inserted thereto, the cosmid vector can accommodate the aforementioned DNA fragment of approximately 34 kb. The aforementioned cosmid sequence has recombinase recognition sequences at both ends thereof. The type of the recognition sequences is determined in accordance with the type of the recombinase in use. For example, the loxP sequence can be used when Cre recombinase is used, and the FRT sequence can be used when FLP recombinase is used. Specifically, the loxP sequence is a sequence of 34 bp and can be used by cutting it out from a known clone (e.g., pBS 246, which was used in the Examples, manufactured by GIBCO BRL co. and other companies). Further, the FRT sequence can be used by cutting it out from the known clone pNEOβGAL (manufactured by Stratagene co. and other companies). Yet further, it is preferable that a DNA sequence having the cloning site (the restriction enzyme site) at least one site other than the adenovirus genome DNA connection site is extended at the outer side of the recombinase recognition sequences of the cosmid sequence.

In the aforementioned method for constructing adenovirus vector of the present invention, a recombinant cosmid/adenovirus vector is prepared by inserting and ligating the expression cassette containing a foreign gene into the circular cosmid/adenovirus vector prepared as described above. The expression cassette is a DNA fragment comprises a promoter sequence, a foreign gene (cDNA) and poly(A) signal and the like.

It should be noted that, in the methods of the first and the second aspects of the present invention, a recombinant cosmid/adenovirus vector may be produced by first connecting the cosmid vector sequence with the expression cassette and then ligating this structure with the adenovirus DNA fragment. Alternatively, a recombinant cosmid/adenovirus vector may be produced by first producing a cosmid/adenovirus vector having an expression cassette inserted thereto, the expression cassette not including any foreign gene (cDNA), and then incorporating a foreign gene (cDNA) into the expression cassette.

In the method according to the first aspect of the present invention, the recombinant cosmid/adenovirus vector prepared as described above is then cotransfected, together with the recombinase-expression vector, to a cell producing the adenovirus E1 protein. As the recombinase-expression vector, the Cre recombinase- or the FLP recombinase-expression vector, for example, may be used. The Cre recombinase recognizes the loxP sequence and cuts out the DNA sequence interposed by two loxP sequences (Nucleic Acids Res. 17:147-161, 1989). The FLP recombinase recognizes the FRT sequence and cuts out the DNA sequence interposed by two FRT sequences (Teends Genet. 9:413-421, 1993). Accordingly, when the recombinase expression vector as described above and the aforementioned recombinant cosmid/adenovirus vector are altogether introduced into a host cell, the cosmid vector sequence interposed by the loxP sequences or the FRT sequences of the circular DNA is cut out by the action of the recombinase, whereby a recombinant adenovirus vector having a linear DNA sequence comprising the adenovirus genome DNA and the expression cassette is constructed.

As the Cre recombinase-expression vector, any suitable known clone (such as MC1-cre plasmid employed in the Examples) may be used. As the FLP recombinase-expression vector, pOG44 (manufactured by Stratagene co.) or the like may be used.

As the cell producing adenovirus E1 protein, the 293 cell line derived from human fetal kidney cells can be preferably used. However, the present invention is not limited to this example, and HeLa cells, normal cells or the like to which the E1 protein gene has been introduced in advance may be used. Further, a cell isolated from a transgenic animal to which the E1 protein gene has been introduced may be used.

On the other hand, in the construction method according to the second aspect of the present invention, the recombinant cosmid/adenovirus vector constructed as described above is transfected to a cell producing recombinase and adenovirus E1 protein. Specifically, in this method, as the host cell stably produces the recombinase, transfecting the host cell only with the recombinant cosmid/adenovirus vector effects, by the action of the recombinase produced by the host cell, cutting out of the cosmid vector sequence interposed by the recombinase recognition sequences of the circular DNA, whereby a recombinant adenovirus vector having a linear DNA sequence comprising the adenovirus genome DNA and the expression cassette is constructed. As the cell producing recombinase and E1 protein, the Cre recombinase producing 293 cell line known by the references (J. Virol. 71:1842-1849, 1997; Proc. Natl. Acad. Sci. USA, 93: 13565-13570, 1996) or the Cre recombinase producing 293 cell line constructed by the method in the Examples described below may be employed. In addition, the FLP recombinase producing 293 cell line which is provided, as a novel product, according to the fourth aspect of the present invention may also be employed. Further, HeLa cells or normal cells to which the E1 protein gene and the recombinase gene have been introduced in advance, or a cell isolated from a transgenic animal to which the E1 protein gene and the recombinase gene have been introduced, may be used.

The whole length of the recombinant adenovirus vector constructed in the aforementioned manner is approximately 36 kb, which is substantially the same as that of the wild type adenovirus. The recombinant adenovirus vector of the present invention is highly infectious to cells of various animals.

The 293 cell line producing FLP recombinase of the fourth aspect of the present invention can be constructed according to any known method. For example, a 293 cell line producing FLP recombinase can be constructed by first incorporating a DNA fragment which encodes the FLP recombinase into a mammalian expression vector, and then introducing the obtained vector to the 293 cell line. The DNA fragment which encodes the FLP recombinase can be cut out, for use, from the known expression vector such as pOG44 (manufactured by Stratagene co.). As the mammalian expression vector, any suitable known vector having a promoter, a splicing region, a poly(A) additional site and the like may be used, in addition to the aforementioned vector pOG44 which can be directly used. For introducing the expression vector into the 293 cell line, any suitable known method such as the electroporation method, the calcium phosphate method, the liposome method and the DEAE dextran method can be employed. Further, a drug resistance gene (such as puromycin resistance gene) may also be incorporated into the FLP recombinase expression vector, so that such a gene functions as the selection marker of the cells to which the vector has been introduced.

Hereinafter, the present invention will be described in detail and more specifically by the following Examples. However, it should be noted that the present invention is not restricted to any of these Examples.

### Example 1

### Construction of a cosmid/adenovirus vector

The cosmid vector SuperCos 1 was purchased from Stratagene co. (La Jolla, CA) and the plasmid pBS246 containing the loxP sequence was purchased from GIBCO BRL co. (Rockville, MD). The Hind III site in the region interposed by the loxP sites of pBS246 was changed to Xba I, and the BamH I site in the same region was changed to Swa I. The resulting plasmid was cut at Not I, so that a fragment of 260 bp having two loxP sites was isolated. This fragment was inserted into the Not I site of the SuperCos 1 whose unique Xba I site had been disrupted in advance, whereby the aimed cosmid vector (pLC cosmid) was prepared.

The adenovirus genome DNA was prepared from pFG 140. The pFG 140 is a plasmid in which the whole genome of the adenovirus 5dl309 is circularized and has a plasmid vector portion of 2.2 kb at the Xba I site in the E1 region thereof. The E1 region (681-2559 bp: counted from the genome 5' end) was deleted by Bal31 nuclease, and an Xba I site was re-constructed at the site of deletion.

The adenovirus DNA whose E1 region had been deleted and the aforementioned pLC cosmid were cut at the Xba I site, respectively, and then ligated. The product was accommodated in the headof bacteriophage λ by the "in vitro" packaging, whereby a cosmid/adenovirus vector of 41 kb (pALC cosmid) was prepared (refer to Fig. 1).

### Example 2

### Construction of a recombinant cosmid/adenovirus vector

On the downstream side of the CAG promoter (the hybrid of the immediate-early enhancer of cytomegalovirus and the chicken β actin promoter) which is known to be highly active in cells of various types, mouse interleukin 5 (mIL-5) cDNA, IRES (Internal ribosome entry site) derived from EMC virus and EGFP (enhanced green fluorescent protein) cDNA derived from pEGFP-NI plasmid were sequentially ligated. Then the Swa I site was added to both ends of the sequence, whereby a DNA fragment was prepared. This DNA fragment was incorporated into a vector derived from pHSG298 having resistance to kanamycin, and the Swa I fragment was cut out from the recombinant vector, whereby the expression cassette (4.2 kb) was prepared.

The expression cassette was inserted into the Swa I site of the pALC cosmid constructed in example 1. Thereafter, the product was incorporated into the headof bacteriophage λ by the "in vitro" packaging, whereby a recombinant cosmid/adenovirus vector (pALC-IL-5) was prepared. The obtained vector was amplified on a large scale in Escherichia coli DH10B, purified and then retrieved.

### Example 3

### Construction of the recombinant adenovirus vector

The recombinant cosmid/adenovirus vector (pALC-IL-5) constructed in example 2 and the Cre recombinase-expression vector (MC1-cre plasmid) were cotransfected to the 293 cell line derived from human fetal kidney cells which stably produce adenovirus E1 protein. MEM (Minimum Essential Medium) containing inactivated 10 % fetal bovine serum (FBS) was used as the culture medium of the 293 cells. The cells were cultured on a petri dish or a multi-plate whose surface had been coated with gelatin.
One µg of pALC-IL-5 and 0.1 µg of MC1-cre plasmid were cotransfected to the 293 cell line cultured in a 12-well multi-plate, using LIPOFECTAMINE (manufactured by GIBCO BRL co.) (see Fig. 2).

The cytopathic effect (CPE), which can be evaluated by the degree of plaque formation, was observed within 10 days after the cotransfection. The culture fluid of the plate in which CPE was observed was centrifuged at 2,000 rpm for 5 minutes at 4 °C, and the supernatant obtained by the centrifuge was added to the 293 cell line cultured in a flask (having the culture surface of 75 cm²) so that infection was effected. After a few days, the cells were collected from the flasks in which CPE was confirmed all over the culture surface. The collected cells were subjected to the freezing-and-thawing process six times and then centrifuged at 3,500 rpm for 10 minutes at 4 °C. Thereafter, the titer of the recombinant adenovirus vector contained in the obtained supernatant was measured. The measurement was carried out by: adding a virus suspension which had been diluted in the serial manner to the 293 cell line cultured on a gelatin-coated 96-well multi-plate; incubating the sample; observing presence/absence of CPE; and calculating the titer from the observed results.

In addition, as a control, only pALC-IL-5 was transfected to the 293 cell line and the plaque formation was examined.

The obtained results were as follows. In the case of the control in which the Cre recombinase was not present, no plaque was observed in any of the 293 cells cultured in the eight wells to which the transfection was performed separately. This result indicates that, as the length of pALC-IL-5DNA is 45 kb and exceeds the length that an infectious adenovirus particle can accommodate, no virus was produced. On the contrary, in the case in which pALC-IL-5DNA was transfected together with the Cre recombinase-expression vector, CPE was observed, within eight days, in all of the 293 cells planted in the eight wells. Additional experiments were carried out repeatedly for each of the control and the cotransfection cases. As a result, in all of the twenty-two wells in which the Cre recombinase co-existed with pALC-IL-5DNA, the plaque was observed.

From the aforementioned results, it is confirmed that the cosmid sequence of pALC-IL-5 was efficiently removed by the Cre recombinase which temporarily expressed itself and, from the circular adenovirus vector genome of 38 kb containing the expression cassette, the infectious recombinant adenovirus vector was produced.

### Example 4

### Analysis on virus DNA

In order to confirm whether or not the cosmid sequence had been removed from the recombinant adenovirus vector constructed in example 3, the structure of DNA thereof was analyzed.

The virus suspension was digested with 1 % SDS, 0.2 mg/ml proteinase K and 10 mM EDTA at 37 °C for 3 hours. The digested sample was then subjected to phenol extraction, phenol/chloroform extraction and ethanol precipitation. The sample was then subjected to electrophoresis in agarose gel, and the virus DNA was extracted from the gel and purified. The virus DNA was digested at Xba I and then subjected to electrophoresis by using 0.4 % and 0.9 % agarose gel.

Fig. 3A shows the structure of the recombinant adenovirus vector genome and the Xba I site therein. The virus DNA purified from the suspension of the recombinant adenovirus vector was digested at the Xba I site and then subjected to electrophoresis in 0.4 % agarose gel (Fig. 3B, lane 1 and lane 2) or in 0.9 % gel (Fig. 3C, lane 1 and lane 2). The virus DNA of lane 1 and that of lane 2 were each prepared from the cell groups which had been subjected to the transfection process independently. Thereafter, the pALC-IL-5DNA was digested at the Xba I site and subjected to electrophoresis in a similar manner. The Xba I fragment containing the cosmid sequence interposed by the loxP sequences is identified as a fragment of 9.1 kb (Figs. 3B and 3C, lane 4). On the other hand, the Xba I fragment contained in the DNA of the recombinant adenovirus vector is recognized as a fragment of 2.1 kb (Fig. 3C, lane 1 and lane 2). The difference between the two fragments corresponds to the cosmid sequence interposed by the loxP in pALC (approximately 7kb). From this result, it is confirmed that the cosmid sequence in pALC-IL-5 was efficiently removed by the transient expression of the Cre recombinase, and the circular adenovirus genome of 38 kb containing the expression cassette which had been formed as a result was amplified and accommodated in virus particles, whereby the infectious recombinant adenovirus vector was produced. No DNA fragment whose size is beyond the expected range was observed. Accordingly, it is also confirmed that abnormal recombination hardly occurs in the recombinant adenovirus vector constructing process according to this method. In the case of the control in which DNA of the 293 cell line, to which the clone pFG140 including the adenovirus genome had been transfected, was digested at the Xba I site, each of the expected fragments of 34.6 kb, 2.2 kb and 1.3 kb was observed (Figs. 3B and 3C, lane 3).

### Example 5

### Expression of the gene contained in the expression cassette

In order to confirm the expression of IL-5, the culture liquid of the infected cells was retrieved and centrifuged, and the IL-5 activity in the obtained supernatant was investigated by the ELISA method. In addition, the expression of EGFP was investigated by the observation with a fluorescent microscope.

In the case in which the recombinant cosmid adenovirus vector pALC-IL-5 and pMC1-cre were cotransfected to the 293 cell line, a relatively large number of cells became EGFP positive, and as the plaque of the virus developed, a strong EGFP fluorescent light mission was observed in the vicinities of all of the plaques (Figs. 4A and 4C).

Next, the suspension of the recombinant adenovirus vector constructed in example 3 was diluted, and the diluted virus vector was infected to the 293 cell line, whereby the plaque was formed. More than 30 plaques were studied, and it was confirmed that all of the plaques were surrounded by EGFP positive cells (Figs. 4B and 4D). Considering that IL-5 and EGFP are transcribed in a bicistronic manner by the CAG promoter, it is expected that the adenovirus vector which expresses EGFP will also express IL-5. Therefore, in order to confirm the expression of IL-5, the virus suspension which had been diluted by the limiting dilution method was added to the 293 cell line cultured in a 96-well multi-plate and virus clones were grown separately. In the case in which ten virus clones were studied, all of the samples were EGFP positive. The IL-5 activity in the supernatant of the culture liquid was studied by using the ELISA method. The results each showed a high value (> 10 µg/ml).

The results described above suggests that the method of the present invention enables production of recombinant adenovirus vector of extremely homogeneous quality, as well as reliable expression, by this vector, of the gene introduced into a mammalian cell.

### Example 6

### Preparation of a 293 cell line producing the Cre recombinase

The Cre recombinase gene and the IRES (internal ribosome entry site) sequence derived from the EMC virus were inserted, together with the puromycin resistance gene, into the EcoR I site of pCAGGS (Gene 108 (2): 193-199, 1991), whereby the Cre recombinase-expression plasmid pCAGGS-cre-puro was constructed. The obtained plasmid was transfected to the 293 cell line, which was cultured in a medium containing puromycin at 2 µg/ml. The Cre recombinase activity was tested by isolating the puromycin resistant colonies and transfecting the 293 cell line with the reporter plasmid pCAG-CAT-Z. Specifically, this reporter plasmid, in which chloramphenicol acetyl transferase (CAT) gene interposed by the loxP sequences is inserted between the CAG promoter and the lacZ gene, causes the expression of lacZ when the CAT gene is cut out by the action of the Cre recombinase, and thus can be used for testing the Cre recombinase production of the 293 cell line (Biochem. Biophys. Res. Commun. 17(2):393-401, 1995). By this test, a cell clone 293cre15 capable of producing the Cre recombinase was obtained.

### Example 7

### Construction of a recombinant adenovirus vector in the 293cre15

One µg/well of the recombinant cosmid/adenovirus vector (pALC-IL-5) constructed in example 2 was transfected to the 293cre15 prepared in example 6. All of the transfected cells in the nine wells showed CPE, and strong EGFP was observed at all of the spots of CPE.

From the results described above, it is confirmed that, by using the 293 cell line producing the recombinase, the recombinant adenovirus vector can be efficiently constructed from the recombinant cosmid/adenovirus vector, without employing the recombinase expression vector.

### Example 8

### Preparation of 293 cell line producing the FLP recombinase

The FLP recombinase gene and the IRES (internal rebosome entry site) sequence derived from the EMC virus were inserted, together with the puromycin resistance gene, into the downstream of the pgk (phosphoglycerate kinase) promoter, whereby the FLP recombinase-expression plasmid pgk-FLP-puro was constructed. The obtained plasmid was transfected to the 293 cell line, which was cultured in a medium containing puromycin at 2 µg/ml. The FLP recombinase activity was tested by isolating the puromycin resistant colonies and transfecting the 293 cell line with the reporter plasmid pNEOβGAL. Specifically, this reporter plasmid, in which the neomycin resistance gene interposed by the FRT sequences is inserted midway of the lacZ gene located on the downstream side of the SV40 promoter, causes the expression of lacZ when the neomycin resistance gene is cut out by the action of the FLP recombinase, and thus can be used for testing the FLP recombinase production of the 293 cell line. By this test, a cell clone 293FLP capable of producing the FLP recombinase was obtained.

### Industrial applicability

As described above in detail, the present invention allows easily and efficiently producing a recombinant adenovirus vector which is infectious to a mammalian cell. In the conventional method of constructing a recombinant adenovirus vector by using homologous recombination, there is a risk that a recombinant vector of unexpected type is produced and the production process thereof is complicated and time-consuming. On the contrary, the method of the present invention does not necessitate such complicated processes and allows reliable production of a recombinant vector having desired structure and function. Further, the method of the present invention allows production of a recombinant adenovirus vector by using a 293 cell line which has been used over no less than seventy passages, while the conventional method employing homologous recombination does not allow employing a 293 cell line which has been used more than 50 passages.

Accordingly, by the method and material provided by the present invention, the analysis of gene function by using a mammalian cell can be facilitated. In addition, the development of a vector used for gene therapy can also be facilitated.

## Claims

1. A method for constructing a recombinant adenovirus victor having a DNA sequence consisting of an adenovirus genome DNA and an expression cassette, which comprises:
constructing a recombinant cosmid/adenovirus vector by inserting and ligating a cosmid sequence having recombinase recognition sequences at both ends and the expression cassette into a site of the adenovirus genome DNA where E1 region or E1 and E3 regions are deleted;
cotransfecting this recombinant cosmid/adenovirus vector and a recombinase-expression vector into a cell line producing adenovirus E1 protein; and
deleting the cosmid vector sequence from the recombinant cosmid/adenovirus vector in the cells.

2. The method according to claim 1, wherein the recombinase is Cre recombinase and the recognition sequences thereof are loxP sequences.

3. The method according to claim 1, wherein the recombinase is FLP recombinase and the recognition sequences thereof are FRT sequences.

4. The method according to any of claims 1 to 3, wherein the cell line producing adenovirus E1 protein is 293 cell derived from human fetal kidney cells.

5. A method for constructing a recombinant adenovirus victor having a DNA sequence consisting of an adenovirus genome DNA and an expression cassette, which comprises:
constructing a recombinant cosmid/adenovirus vector by inserting and ligating a cosmid sequence having recombinase recognition sequences at both ends and the expression cassette into a site of the adenovirus genome DNA where E1 region or E1 and E3 regions are deleted;
transfecting this recombinant cosmid/adenovirus vector into a cell line producing recombinase and adenovirus E1 protein; and
deleting the cosmid vector sequence from the recombinant cosmid/adenovirus vector in the cells.

6. The method according to claim 5, wherein the recombinase is Cre recombinase and the recognition sequences thereof are loxP sequences.

7. The method according to claim 5, wherein the recombinase is FLP recombinase and the recognition sequences thereof are FRT sequences.

8. The method according to any of claims 5 to 7, wherein the cell line producing recombinase and adenovirus E1 protein is 293 cell derived from human fetal kidney cells which produces the recombinase.

9. A cosmid/adenovirus vector, which comprises a cosmid sequence having recombinase recognition sequences at both ends in a site of the adenovirus genome DNA where E1 region or E1 and E3 regions are deleted.

10. The cosmid/adenovirus vector of claim 9, wherein the recombinase is Cre recombinase and the recognition sequences thereof are loxP sequences.

11. The cosmid/adenovirus vector of claim 9, wherein the recombinase is FLP recombinase and the recognition sequences thereof are FRT sequences.

12. A 293 cell line derived from human fetal kidney cells, which produces FLP reccombinase.
